# EUROPEAN PATENT SPECIFICATION

(11) **EP 0 985 006 B1**
(45) Date of publication and mention of the grant of the patent: **03.08.2005**
(21) Application number: 98921382.2
(22) Date of filing: 29.05.1998
(51) Int. Cl.: C09J 167/00, C09J 123/00, C09J 125/08

(54) **AN ADHESIVE AGENT AND USE OF SUCH ADHESIVE AGENT**
KLEBEMITTEL UND VERWENDUNG DIESES KLEBEMITTELS
AGENT ADHESIF ET UTILISATION DE CET AGENT ADHESIF

(30) Priority: 30.05.1997 DK 63697
(43) Date of publication of application: 15.03.2000
(73) Proprietor: COLOPLAST A/S, 3050 Humlebaek (DK)
(72) Inventor: NIELSEN, Inger, Mann, DK-2000 Frederiksberg (DK); NIELSEN, Anders, Christian, DK-2200 Koebenhavn N (DK); CIOK, Danuta, DK-2990 Nivaa (DK)
(74) Representative: Nilausen, Kim
(86) International application number: PCT/DK1998/000229
(87) International publication number: WO 1998/054269

(56) References cited:
- EP-A- 0 781 538
- WO-A-95/18191
- US-A- 4 551 490
- US-A- 5 512 612

## Description

### FIELD OF THE INVENTION

The invention relates to a pressure sensitive adhesive composition suitable for application to human or animal skin, to a method for preparing such adhesives and the use of such adhesive for the preparation of a wound dressing or a adhesive wafer for an ostomy appliance or the use of the adhesive agent for securing of and sealing around ostomy bandages, for securing wound dressings, for securing of devices for collecting urine, wound-drainage bandages, orthoses and prostheses and for protection skin areas and parts of the body against pressure, impacts and friction and to wound dressings or ostomy appliances comprising such adhesive composition.

### BACKGROUND OF THE INVENTION

Various skin adhesive agents are used today for the above mentioned purposes.

A very widespread embodiment of skin adhesive agents comprises a self-adhesive elastomeric matrix, in which water-absorbing, swelling particles, the so-called hydrocolloids, are dispersed.

Adhesive compositions comprising hydrocolloids have been known for many years. US. patent No. 3,339,549 discloses a blend of a rubbery elastomer such as polyisobutylene and one or more water soluble or water swellable hydrocolloids such as a powdery mixture of pectin, gelatine and carboxymethylcellulose. The adhesive mass has a water-insoluble film applied to one surface. A composition of this type is available commercially from E.R. Squibb & Sons Inc. under the trademark "Stomadhesive" and is used as a skin barrier around stomas to prevent skin breakdown by the corrosive fluids discharged by the stoma and is also used for adhesive wafers for ostomy appliances.

In adhesive compositions of this type, the polyisobutylene is responsible for provision of the adhesive properties and the dispersed hydrocolloid powders absorb fluid and render the adhesive agent capable of also adhering to moist skin (wet tack). These compositions are also gaining increasing acceptance as wound dressings for dermal ulcers, burns and other exuding wounds.

One major problem which has been encountered with conventional adhesive compositions comprising hydrocolloids is their susceptibility to breakdown upon exposure to body fluids. When the compositions are used as skin barriers, e.g., around stomas, absorption of fluid is desirable, but excessive swelling of the hydrocolloids upon absorption causes the composition to lose its integrity opening for leaks and the barrier must be replaced more often than is desirable from a skin protection point of view, and very often, a residue remains on the skin, which in many cases is difficulty to remove.

When bandaging wounds, contact with the wound exudate will in a similar way effect a disintegration of the adhesive agent which means that when the bandage is changed remnants will be left in the wound, which remnants may affect the wound-healing process. Besides during use leakage may arise which partly means reduced time of use, partly may increase the risk of contaminating the wound with bacteria or other microorganisms.

Adhesive agents are also used for securing devices, such as uridomas, for collecting the urine from incontinent men. Disintegration of the adhesive agent due to contact with urine will again mean a risk of leakage and a reduction of the time of use.

Adhesive agents are also employed for securing orthoses and prostheses (e.g. breast prostheses) and for protection of skin areas or parts of the body against pressure, impact and friction. In these cases it is primarily the secretion of sweat which may cause swelling and disintegration of the adhesive agent. When removing the adhesive agent remnants will be left on the skin, involving the inconveniences earlier mentioned.

A number of attempts have been made to improve the integrity of adhesive compositions.

As a method for improving the adhesive integrity the use of hydrocolloids has been described which, in themselves, are cross-linked (e.g. cross-linked carboxymethylcellulose (CMC), cross-linked dextrane and other water-absorbing, but insoluble hydrocolloids). They will not dissolve due to the cross-linked structure. During the swelling process the individual particles will, therefore, obtain a gel-like structure, but no coherent gel could be formed since the macromolecules of the cross-linked hydrocolloids are locked in the gel network constituted by the individual particles. Due to the lack of a coherent gel, the cross-linked hydrocolloids will be leached out and suspended in the body fluids and the effect on the integrity of the swelled adhesive, therefore, is limited.

Alternatively, it has been tried to increase the integrity of the swelled adhesive agent by increasing the cohesion of the elastomeric phase (as described in several of the prior art patents referred to). The elastomeric phase, therefore, will not so easily be split by the expanding hydrocolloids during the swelling process. This process, however, has a number of drawbacks:
- The rate of water absorption and thus the "wet tack" of the adhesive agent will be reduced.
- By strengthening the cohesion the elastomeric matrix will have stronger elastic properties. When the hydrocolloids absorb water and swell, this will enhance an increase in the dimensions of the adhesive agent. Due to the elastic properties of the matrix, the tensions occurring in the adhesive agent cannot be relaxed by plastic deformation. Instead pleats may occur in the adhesive agent around the swelled area. In these pleats the adhesive agent will loose contact with the skin exposed to the body fluids, and a risk of leakage arises.

US. Patent Nos. 4,192,785 and 4,551,490 describe incorporating into an adhesive composition of a cohesive strengthening agent such as a natural or synthetic fibrous material, finely divided cellulose, cross-linked dextran, cross-linked carboxymethylcellulose or a starch-acrylonitrile graft copolymer. The cohesive strengthening agent is stated to control the rate of hydration of the composition thereby increasing the resistance against breakdown by body fluids.

US. Patent No. 4,477,325 describes incorporation of a mixture of a copolymer resin of ethylene and vinyl acetate (EVA) into the adhesive composition. After mixing and moulding, the composition is subjected to ionising radiation to form a cross-linked polymer network of the EVA or comprising EVA and another cross-linked resin. The cross-linked matrix is said to provide a controlled swelling.

US. Patent No. 4,496,357 describes the incorporation of fumed silica into adhesive compositions to control swelling.

EP No. 0 122 344 B1 describes incorporation of one or more natural or synthetic polymers capable of developing elastomeric properties when hydrated, such as gluten and long chain polymers of methyl vinyl ether/maleic acid, into the adhesive composition. The adhesive composition is stated to be resistant to erosion by moisture and body fluids.

EP patent No. 0 340 945 B1 describes incorporation of some polycationic hydrocolloid particles into a hydrocolloid composition. The mixture of polycationic, polyanionic and neutral hydrocolloids is stated to provide increased integrity without a concomitant decrease in absorbing capacity.

WO 95/18191 A1 discloses a water-dispersible adhesive composition, in particular a hot-melt adhesive, including a low molecular weight, branched copolyester containing a sulfomonomer.

US 5,512,612 discloses a pressure sensitive adhesive comprising a water-dispersible polymer, e.g. a polymer comprising poly(alkoxyalkyl)acrylate units.

EP 0 781 538 A (which was filed before, but published after the priority date of the present application) discloses a hot-melt adhesive composition comprising at least one water-dispersible copolyester, a plasticizer, and a compatible tackifier.

Another major problem for conventional adhesive compositions comprising hydrocolloids is their limited capability in adhering to moist body surfaces. There is particularly a need for an improved adhesive composition having an enhanced adhesion to moist skin in the management of ostomy patients, as it is often difficult to keep the skin around stomas completely dry during replacement of an ostomy appliance.

In existing adhesive agents the surface of the adhesive is consisting of the self-adhesive elastomeric matrix while the hydrocolloids are located embedded beneath in the elastomeric matrix. In order to absorb moisture, the water thus needs to penetrate through the elastomeric matrix before reaching the water absorbing hydrocolloids. This retards the water-absorption and causes that the adhesive agent does not have an immediately adhesion to wet surfaces (wet tack).

Skin problems associated with an ostomy are differing from skin problems generally associated with adhesives for skin (dressings or plasters) as the adhesives of ostomy appliances are placed permanently at the same site during long periods of time (cronical irritation) whereas other adhesives for skin are normally only placed at the same site for a short period of time.

European Patent publication No. EP 0 017 401 A1 discloses articles of manufacture having adhesive properties useful for, for example, protective plasters or dressings or as rings, washers or the like in surgical appliances such as ostomy appliances comprising a plastics matrix comprising the product resulting from heating together one or more polyhydric alcohols and gelatine and/or naturally occurring high molecular weight polysaccharide gum and/or a resin which is a copolymer and a vinyl ether and an organic acid anhydride and/or its corresponding free acid. Polyvinylpyrrolidone resin may be added as a tack modifier.

European Patent publication No. EP 0 343 807 A2 discloses absorptive adhesive dressing with controlled hydration containing about 30-65% polyisobutylene, 10-30% polyvinylpyrrolidone, 2-20% modified starch, 2-20% pectin, 0.1-10% acrylic polymer and 0-1% fibre.

European Patent publication No. EP 0 063 898 discloses a microporous tape comprising a porous backing layer and a microporous adhesive layer including a rubbery elastomer such as polyisobutylene, one or more water swellable hydrocolloids and other optional substances. A copolymer of polyvinylpyrrolidone and vinylacetate may be used as a tackifier.

Thus, there is still a need for an adhesive composition showing a very rapid water absorption and retention in order to improve wet tack and which is, at the same time, resistant to disintegration upon contact with body fluids.

It has surprisingly been found that adhesive compositions comprising a water-dispersible polyester shows a very rapid water absorption and improves the wet tack and is, at the same time, resistant to disintegration upon contact with body fluids.

### BRIEF DESCRIPTION OF THE INVENTION

The present invention relates to a pressure sensitive adhesive composition suitable for application to human or animal skin comprising a water dispersible polyester.

The invention also relates to a method for preparing a pressure sensitive adhesive composition suitable for application to human or animal skin comprising a water dispersible polyester and to the use of such adhesive for the preparation of a wound dressing or a adhesive wafer for an ostomy appliance or the use of the adhesive agent for securing of and sealing around ostomy bandages, for securing wound dressings, for securing of devices for collecting urine, wound-drainage bandages, orthoses and prostheses and for protection skin areas and parts of the body against pressure, impacts and friction and to wound dressings or ostomy appliances comprising such adhesive composition.

### DETAILED DESCRIPTION OF THE INVENTION

The present invention relates to a pressure sensitive adhesive composition suitable for application to human or animal skin comprising a) a water dispersible polyester, b) one or more polymer(s) selected from the group consisting of polyolefins or copolymers or blends thereof, and/or one or more physically cross-linked elastomers selected from block-copolymers comprising styrene and one or more olefins, and/or one or more polyethylene glycols and/or glycerol; c) optionally a butene polymer or copolymer, d) optionally a tackifying resin which may be partly or totally hydrogenated, e) optionally a microcrystalline wax, f) optionally a plasticizer, g) one or more hydrocolloids and h) optionally a pigment.

By introducing a water dispersible polyester in a self-adhesive elastomeric matrix an improved adhesion in moist environment is achieved. This is ascribed to the fact that the water dispersible polyester is present at the surface of the adhesive agent and thus is able to cause an immediately absorption of water. The water-absorbing polymer furthermore remains sticky after having absorbed water which is in contrast to the non-absorbing elastomers used in conventional skin friendly adhesives and hence, a better adhesion over longer periods of time is obtained.

The pressure sensitive adhesive composition of the invention typically comprises 10-50% of a water dispersible polyester, 10-70% of one or more polymer(s) selected from the group consisting of polyolefins or copolymers or blends thereof, and/or one or more physically cross-linked elastomers selected from block-copolymers comprising styrene and one or more olefins, and/or one or more polyethylene glycols and/or glycerol; 0-15% of a butene polymer or copolymer, 0-30% of a tackifying resin which may be partly or totally hydrogenated, 0-10% of a microcrystalline wax, 0-10% of a plasticizer, up to 50% of one or more hydrocolloids and 0-5% of a pigment.

The water dispersible polyester is preferably a water-dispersible branched polyester.

The use of branched water-dispersible polyester in adhesive agents gives the following advantages:
- The adhesive agent shows a superior adhesion in moist environments by having an immediately absorption of water giving a better wet tack as compared to conventional adhesive agents based on non-absorbing self-adhesive elastomers. The branched water-dispersible polyester is also contributing by giving a good adherence over a longer periods of time since the stickiness of the elastomer is retained even after absorption of body fluids.
- The adhesive agent is easily removed by washing due to the water-dispersibility of the polyester in tap water, but the adhesive agent is not affected by body fluids. The body fluids are ionic solutions in which the polyester component is not dispersible. Adhesive residues left on the skin can thus be removed very easily using tap water.

Suitable water-dispersible branched polyester are polyesters having the following physical properties: a Brookfield Thermosel viscosity at 177°C of 1,000-500,000 mPa.s, more preferred 1,000-50,000 mPa.s, and preferably of 2,000-8,000, a ring & ball softening point of 50-150°C more preferred of 80-90°C, a penetration hardness as determined according to ASTM D 5 of 5-50 dmm, preferably about 30 dmm and a glass transition temperature T_{g} according to ASTM D 3418 of from 5 to -10 °C, preferably about -5°C

A reduction of the amount of hydrocolloids is rendered possible as the branched water-dispersible polyester contributes to the absorption. A reduction of the amount of hydrocolloids improves the stability of the adhesive due to the reduction of the disintegration caused by the considerable swelling of the hydrocolloids in conventional skin friendly adhesives upon absorption of body fluids.

Suitable hydrocolloids are naturally occurring hydrocolloids such as guar, locust bean gum (LBG), pectin, alginates, gelatine, xanthan or karaya, semisynthetic hydrocolloids such as cellulose derivatives (e.g. salts of carboxymethylcellulose, methylcellulose and hydroxypropylmethylcellulose), sodium starch glycolate and synthetic hydrocolloids such as polyvinylalcohol, polyethylene glycol or polyacrylates.

Pigments may be used as fillers or for dying the adhesives of the invention. Suitable pigments are pigments conventionally used in adhesive compositions, e.g. titanium dioxide or zinc oxide. Zinc oxide offers the advantage of showing an antibacterial effect.

Preferred adhesives according to the invention are e.g. adhesives comprising branched water-dispersible polyester and amorphous poly-alpha-olefines and having the composition:
0 - 10 % Ethylene/1-butene Copolymer (e.g. Hüls Vestoplast 520)
20 - 40% Propene/1-hexene Copolymer (e.g. Eastman Eastoflex D-127)
0 - 10% Triblock copolymer (e.g. SIS, such as Kraton® D1107 from Shell Chemicals)
0 - 15 % Polybutene (e.g. Hyvis 10, Hyvis 2000)
0 - 15% Liquid polyterpene-tackyfier (e.g. Goodyear Wingtack 10)
0 - 15% Hydrogenated poly(hydrocarbon/terpene) tackyfier (e.g. Goodyear Wingtack 95).
0 - 15% Optionally hydrogenated rosin acid and esters thereof
0 - 10 % Plastisizer (e.g. dioctyl adipate)
10 - 50% Eastman water-dispersible branched polyester (e.g. AQ1045)
up to 50% Hydrocolloids (e.g. CMC, Natrosol, Ca-alginat etc.)
0 - 5% Pigment, e.g. zinc oxide

Other preferred adhesives according to the invention are e.g. adhesives comprising branched water-dispersible polyester and block copolymers and having the composition:
0 - 10 % Triblock copolymer (e.g. SIS)
10 - 50 % Diblock copolymer (e.g. SI)
0 - 10 % Plastisizer (e.g. dioctyladipate)
0 - 15 % Tackifier (e.g. liquid polyterpene, hydrogenated poly(hydrocarbon/terpene)
0 - 10 % Microcrystalline wax (e.g. Wax Total 40/60 from Total)
10 - 50 % Eastman water-dispersible branched polyester (e.g. AQ1045)
up to 50% Hydrocolloids (e.g. CMC, Natrosol, Ca-alginat etc.)
0 - 5% Pigment, e.g. zinc oxide

Further preferred adhesives according to the invention are e.g. adhesives comprising branched water-dispersible polyester and glycerol and having the composition:
10 - 50 % Glycerol
10 - 50 % Eastman water-dispersible branched polyester (e.g. AQ1045)
up to 50% Hydrocolloids (e.g. CMC, Natrasol, Ca-alginat etc.)
0 - 5% Pigment, e.g. zinc oxide

Yet further preferred adhesives according to the invention are e.g. adhesives comprising branched water-dispersible polyester and polyethylene glycol (PEG) and having the composition:
10 - 50 % Polyethylene glycol (e.g. PEG 400)
10 - 50 % Eastman water-dispersible branched polyester (e.g. AQ1045)
up to 50% Hydrocolloids (e.g. CMC, Natrosol, Ca-alginat etc.)
0 - 5% Pigment, e.g. zinc oxide

The olefin component of the block-copolymers comprising styrene and one or more olefins is preferably a diene, especially a polybutadiene or polyisoprene. The olefin may also be a polyisobutylene.

In a second aspect, the invention relates to a method for preparing a pressure sensitive adhesive composition suitable for application to human or animal skin comprising a water dispersible polyester, one or more polymer(s) selected from the group consisting of polyolefins or copolymers or blends thereof, one or more physically cross-linked elastomers selected from block-copolymers comprising styrene and one or more olefins, polyethylene glycols and/or glycerol; optionally a butene polymer or copolymer, optionally a tackifying resin which may be partly or totally hydrogenated, optionally a microcrystalline wax, optionally a plasticizer, one or more hydrocolloids and optionally a pigment, characterised in that the polymer(s) selected from the group consisting of polyolefins or copolymers or blends thereof, one or more physically cross-linked elastomers selected from block-copolymers comprising styrene and one or more olefins, polyethylene glycols and/or glycerol is mixed, whereafter the water dispersible polyester is admixed and mixed until homogeneity, a plasticizer is optionally admixed, and one or more hydrocolloids are admixed under a reduced pressure whereafter the adhesive agent is removed from the mixer and optionally pressed into 1 mm thin plates between two sheets of release paper.

The invention relates to a further method for preparing a pressure sensitive adhesive composition suitable for application to human or animal skin comprising a water dispersible polyester, one or more polymer(s) selected from the group consisting of polyolefins or copolymers or blends thereof, one or more physically cross-linked elastomers selected from block-copolymers comprising styrene and one or more olefins, polyethylene glycols and/or glycerol; optionally a butene polymer or copolymer, optionally a tackifying resin which may be partly or totally hydrogenated, optionally a microcrystalline wax, optionally a plasticizer, one or more hydrocolloids and optionally a pigment, characterised in that the polymer(s) selected from the group consisting of polyolefins or copolymers or blends thereof, one or more physically cross-linked elastomers selected from block-copolymers comprising styrene and one or more olefins, polyethylene glycols and/or glycerol; a butene polymer and/or a tackifying resin which may be partly or totally hydrogenated and/or a microcrystalline wax and/or a plasticizer is(are) optionally admixed and mixed until homogeneity, the water dispersible polyester is admixed and mixed until homogeneity, and one or more hydrocolloids are admixed under a reduced pressure whereafter the adhesive agent is removed from the mixer and optionally pressed into 1 mm thin plates between two sheets of release paper.

In a third aspect the invention relates to the use of a pressure sensitive adhesive composition suitable for application to human or animal skin comprising a water dispersible polyester, one or more polymer(s) selected from the group consisting of polyolefins or copolymers or blends thereof, one or more physically cross-linked elastomers selected from block-copolymers comprising styrene and one or more olefins, polyethylene glycols and/or glycerol; optionally a butene polymer or copolymer, optionally a tackifying resin which may be partly or totally hydrogenated, optionally a microcrystalline wax, optionally a plasticizer, one or more hydrocolloids and optionally a pigment for securing of and sealing around ostomy bandages, for securing wound dressings, for securing of devices for collecting urine, wound-drainage bandages, orthoses and prostheses and for protection skin areas and parts of the body against pressure, impacts and friction.

In a further aspect, the invention relates to the use of a water dispersible polyester for the preparation of a pressure sensitive adhesive composition suitable for application to human or animal skin, said adhesive comprising one or more physically cross-linked elastomers selected from block-copolymers comprising styrene and one or more olefins, polyethylene glycols and glycerol; optionally a butene polymer or copolymer, optionally a tackifying resin which may be partly or totally hydrogenated, optionally a microcrystalline wax, optionally a plasticizer, one or more hydrocolloids and optionally a pigment.

In still further aspects, the invention relates to wound dressings or ostomy appliances comprising an adhesive composition according to the invention. Such wound dressings or ostomy appliances resemble conventional wound dressings or ostomy appliances apart from the substitution of the adhesive of a dressing or ostomy adhesive plate or wafer with an adhesive according to the invention.

The invention is explained more in detail with reference to the below examples disclosing preferred embodiments of the invention. The examples are thus not to be considered limiting of the scope of the invention as set forth in the appended claims.

### MATERIALS AND METHODS

AQ1045 from Eastman. A branched water-dispersible polyester.
AQ1350 from Eastman. A branched water-dispersible polyester.
Kraton® D 1107 from Shell Chemical Company. Styrene-isoprene-styrene copolymer (SIS) having a molecular weight of 212,000-260,000 as determined by GPC.
Vector® 4114 from Exxon: Styrene-isoprene-styrene copolymer (SIS) having a molecular weight of 130,000 and a content of diblock copolymer of 40% and 15% styrene.
LVSI 101 from Shell Chemical Company. Styrene-isoprene diblock copolymer (SI) having a molecular weight of 30,000 as determined by GPC
Dioctyladipate from International Speciality Chemicals Ltd. A plastisizer.
METALYN 200, a methyl ester of rosin from Hercules
Eastoflex E1003, E 1060 and E 1200 from Eastman. propylene-ethylene copolymers.
Eastoflex D127 from Eastman. A propylene/1-hexene copolymer
Vestoplast 704, 708 and 750: amorphous propylene-rich poly-α-olefins from Hüls Chemie
Wingtack 10 from Goodyear. A liquid polyterpene tackyfier resin
Arkon P90: A fully saturated alicyclic light coloured hydrocarbon resin having molecular weight of 650 from ARAKAWA Chemical Industries
Arkon P70: A fully saturated alicyclic light coloured hydrocarbon resin having molecular weight of 570 from ARAKAWA Chemical Industries
Foral 85: Foral 85-E, A hydrogenated Rosin Glycerol Ester from HERCULES Inc.
Foral AX-E: A hydrogenated Rosin from HERCULES Inc.
Glycerol.
PEG 400 from Hoechst. Polyethylene glycol.
Blanose 9H4XF from Hercules. Sodium carboxymethylcellulose.
Pectin USP/100 from Copenhagen Pectin.
Klucel HXF EP. Hydroxypropyl cellulose.
Gelatine P.S.98.240.233 from ED. Geistlich Sohne AG
Zink Oxide Pharma from Hoechst AG.

### EXPERIMENTAL PART

### Preparation of the adhesives according to the invention

### Example 1

An adhesive agent having the composition stated in Table 1 was prepared in a Z-blade mixer. Before the mixing, the mixing chamber was heated to 140°C by means of an oil heater. AQ1045, Eastoflex D127, Eastoflex E1003, dioctyladipate and the hydrocolloids were weighed out separately. Firstly Eastoflex D127 and E1003 were mixed for 15 minutes. AQ1045 was added and the mixing continued for 10 minutes. Dioctyladipate was added and mixed for additional 10 minutes. The heat supply was turned off and the mixing chamber was cooled down to 80°C. The hydrocolloids (a mixture of pectin, hydroxypropyl cellulose and gelatine in the ratio 1:1.5:1) were added and the mixing was continued in vacuo until a total mixing time of 60 minutes. The adhesive agent was removed from the mixer and pressed into 1 mm thin plates between two sheets of silicon paper in a hydraulic press at 90°C.

### Example 3

An adhesive agent having the composition stated in Table 1 was prepared in a Z-blade mixer. Before the mixing, the mixing chamber was heated to 150°C by means of an oil heater. AQ1045, Kraton D1107, LVSI101, dioctyladipate and the hydrocolloids were weighed out separately. Firstly Kraton and dioctyldiadipate were mixed at 150°C for 15 minutes. LVSI 101 was added and the mixing continued for 10 minutes. The mixing chamber was cooled down to 130°C and AQ1045 was added and the mixing was continued for an additional 15 minutes. The heat supply was turned off and the mixing chamber was cooled down to 80°C. The hydrocolloids, a mixture of pectin and hydroxypropylcellulose in the ratio 1:1, and finally zinc oxide were added and the mixing was continued in vacuo until a total mixing time of 60 minutes. The adhesive agent was removed from the mixer and pressed into 1 mm thin plates between two sheets of silicon paper in a hydraulic press at 90°C.

### Example 4

An adhesive agent was having the composition stated in Table 1 prepared in a Z-blade mixer. Before the mixing, the mixing chamber was heated to 130°C by means of an oil heater. AQ1045 and glycerol and the hydrocolloids were weighed out separately. Firstly AQ1045 and glycerol were mixed at 130°C for 15 minutes. The heat supply was turned off and the mixing chamber was cooled down to 80°C. The hydrocolloids, a mixture of pectin and gelatine in the ratio 1:2, were added and the mixing continued in vacuo until a total mixing time of 40 minutes. The adhesive agent was removed from the mixer and is pressed into 1 mm thin plates between two sheets of silicon paper in a hydraulic press at 90°C.

### Example 5

An adhesive agent having the composition stated in Table 1 was prepared in a Z-blade mixer. Before the mixing, the mixing chamber was heated to 130°C by means of an oil heater. AQ1045 and PEG 400 and the hydrocolloids were weighed out separately. Firstly AQ1045 and PEG 400 were mixed at 130°C for 15 minutes. The heat supply was turned off and the mixing chamber was cooled down to 80°C. The hydrocolloids, a mixture of hydroxypropylcellulose and gelatine in the ratio 1:1, were added and the mixing continued in vacuo until a total mixing time of 40 minutes. The adhesive agent was removed from the mixer and pressed into 1 mm thin plates between two sheets of silicon paper in a hydraulic press at 90°C.

### Example 6

An adhesive agent having the composition stated in Table 1 was prepared in a Z-blade mixer in the same manner as described in Example 3.

**Table 1**

| Constituent | Example 1 | Example 3 | Example 4 | Example 5 | Example 6 |
|---|---|---|---|---|---|
| AQ1045 | 35 | 30 | 50 | 40 | 32 |
| AQ 1350 | | | | | |
| LVSI 101 | | 29 | | | 25 |
| Kraton D 1107 | | 5 | | | 5 |
| Doctyladipate | 5 | 5 | | | 8 |
| Eastoflex D127 | 15 | | | | |
| Eastoflex E1003 | 10 | | | | |
| Wingtack 10 | | | | | |
| Glycerol | | | 20 | | |
| PEG 400 | | | | 20 | |
| Blanose 9H4XF | | | | | |
| Pectin USP/100 | 10 | 15 | 10 | | 15 |
| Klucel HXF EP | 15 | 15 | | 20 | 15 |
| Gelatine | 10 | | 20 | 20 | |
| Zinc oxide | | 1 | | | |

### EXAMPLES 7 - 11

Adhesives compositions according to the invention being suitable as pastes having the compositions stated in Table 2 were prepared in a Z-mixer in the same manner as described in Example 1

**Table 2**

| Constituent | Example 7 | Example 8 | Example 9 | Example 10 | Example 11 |
|---|---|---|---|---|---|
| Eastoflex E 1003 | 15 | 15 | 15 | 20 | 20 |
| Eastoflex E 1060 | | | | 2.5 | 2.5 |
| AQ 1350 | 35 | 35 | 35 | 35 | 35 |
| Dioctyladipate | 5 | 5 | 5 | 5 | |
| Metalyn 200 | | | | | 5 |
| Vestoplast 708 | 10 | 5 | 5 | 2.5 | 2.5 |
| Vestoplast 704 | | 5 | 2.5 | | |
| Vestoplast 750 | 10 | | 2.5 | | |
| Gelatine | 10 | 10 | 10 | 10 | 10 |
| Pectin | 15 | 10 | 10 | 10 | 10 |
| Klucel | | 15 | 15 | 15 | 15 |

### EXAMPLES 12 - 21

Adhesive agents having the composition stated in Tables 3-4 were prepared in a Z-blade mixer. Before the mixing, the mixing chamber was heated to 150°C by means of an oil heater. AQ1045, Vector 4114, LVSI 101, Arkon P-90 and the hydrocolloids were weighed out separately. Firstly Vector was mixed at 150°C for 15 minutes. LVSI 101 was added and the mixing continued for 15 minutes. The mixing chamber was cooled to 130°C and Arkon P-90, Arkon P-70, Foral 85-E or Foral AX-E followed by AQ1045 were added and the mixing was continued for an additional 30 minutes. The heat supply was turned off and the mixing chamber was cooled to 80°C. The pectin or Klucel was added and the mixing was continued in vacuo until a total mixing time of 90 minutes. The adhesive agent was removed from the mixer and pressed into 1 mm thin plates between two sheets of silicon paper in a hydraulic press at 90°C.

**Table 3**

| Constituent | Example 12 | Example 13 | Example 14 | Example 15 | Example 16 |
|---|---|---|---|---|---|
| AQ1045 | 30 | 30 | 30 | 25 | 30 |
| LVSI 101 | 25 | 25 | 25 | 20 | 25 |
| Vector® 4114 | 5 | 5 | 5 | 12 | 5 |
| ArkonP90 | 10 | 10 | | 13 | |
| Arkon P70 | | | 10 | | |
| Foral 85-E | | | | | 10 |
| Foral AX-E | | | | | |
| Klucel HXF | 15 | | 15 | 15 | |
| Pektin | 15 | 30 | 15 | 15 | 30 |

**Table 4**

| Constituent | Example 17 | Example 18 | Example 19 | Example 20 | Example 21 |
|---|---|---|---|---|---|
| AQ1045 | 30 | 35 | 35 | 35 | 40 |
| LVSI 101 | 25 | 25 | 30 | 25 | 20 |
| Vector® 4114 | 5 | 5 | 5 | 5 | 5 |
| ArkonP90 | | 15 | 10 | 10 | 10 |
| Arkon P70 | | | | | |
| Foral 85-E | | | | | |
| Foral AX-E | 10 | | | | |
| Klucel HXF | | | | | |
| Pektin | 30 | 20 | 20 | 25 | 25 |

## Claims

1. A pressure sensitive adhesive composition suitable for application to human or animal skin comprising a) a water-dispersible polyester, b) one or more polymer(s) selected from the group consisting of polyolefins or copolymers or blends thereof, and/or one or more physically cross-linked elastomers selected from block-copolymers comprising styrene and one or more olefins, and/or one or more polyethylene glycols and/or glycerol; c) optionally a butene polymer or copolymer, d) optionally a tackifying resin which may be partly or totally hydrogenated, e) optionally a microcrystalline wax, f) optionally a plasticizer, g) one or more hydrocolloids and h) optionally a pigment.

2. An pressure sensitive adhesive composition as claimed in claim 1, **characterised in that** it comprises 10-50% of a water-dispersible polyester, 10-70% of one or more polymer(s) selected from the group consisting of polyolefins or copolymers or blends thereof, and/or one or more physically cross-linked elastomers selected from block-copolymers comprising styrene and one or more olefins, and/or one or more polyethylene glycols and/or glycerol; 0-15% of a butene polymer or copolymer, 0-30% of a tackifying resin which may be partly or totally hydrogenated, 0-10% of a microcrystalline wax, 0-10% of a plasticizer, up to 50% of one or more hydrocolloids and 0-5% of a pigment.

3. The adhesive composition as claimed in claim 1 or 2, **characterised in that** the water dispersible polymer is a water-dispersible branched polyester.

4. The adhesive composition as claimed in claim 3, **characterised in that** the branched polyester has a Brookfield Thermosel viscosity at 177°C of 1,000-500,000.

5. The adhesive composition as claimed in any one of the preceding claims, wherein the one or more hydrocolloids are selected from naturally occurring hydrocolloids, semisynthetic hydrocolloids and synthetic hydrocolloids.

6. The adhesive composition as claimed in claim 5, wherein the one or more hydrocolloids are selected from guar gum, locust bean gum, pectin, alginates, gelatine, xanthan gum, karaya gum, cellulose derivatives, sodium starch glycolate, polyvinylalcohol, polyethylene glycol, and polyacrylates.

7. The adhesive composition as claimed in any one of the preceding claims, which comprises branched water-dispersible polyester and amorphous poly-alpha-olefines and has the composition:
0-10 % ethylene/1-butene Copolymer;
20-40% propene/1-hexene Copolymer;
0-10% triblock copolymer;
0-15 % polybutene;
0-15% liquid polyterpene-tackyfier;
0-15% hydrogenated poly(hydrocarbon/terpene) tackyfier;
0-15% optionally hydrogenated rosin acid and esters thereof;
0-10% plastisizer;
10-50% Eastman water-dispersible branched polyester;
up to 50% hydrocolloids; and
0-5% pigment.

8. The adhesive composition as claimed in any one of the claims 1-6, which comprises branched water-dispersible polyester and block copolymers and has the composition:
0-10% triblock copolymer;
10-50% diblock copolymer;
0-10% plastisizer;
0-15% tackifier;
0-10% microcrystalline wax;
10-50% Eastman water-dispersible branched polyester;
up to 50% hydrocolloids; and
0-5% pigment.

9. The adhesive composition as claimed in any one of the claims 1-6, which comprises branched water-dispersible polyester and glycerol and has the composition:
10-50% glycerol;
10-50% Eastman water-dispersible branched polyester;
up to 50% hydrocolloids; and
0-5% pigment.

10. The adhesive composition as claimed in any one of the claims 1-6, which comprises branched water-dispersible polyester and polyethylene glycol and having the composition:
10-50% Polyethylene glycol;
10-50% Eastman water-dispersible branched polyester;
up to 50% hydrocolloids; and
0-5% pigment.

11. A method for preparing a pressure sensitive adhesive composition as defined in any one of the claims 1-10, **characterised in that** the one or more polymers are mixed, whereafter the water dispersible polyester is admixed and mixed until homogeneity, a plasticizer is optionally admixed, and one or more hydrocolloids are admixed under a reduced pressure, whereafter the adhesive agent is removed from the mixer and optionally pressed into 1 mm thin plates between two sheets of release paper.

12. Use of a pressure sensitive adhesive composition as defined in any one of claims 1-10 for securing of and sealing around ostomy bandages, for securing wound dressings, for securing of devices for collecting urine, wound-drainage bandages, orthoses and prostheses and for protection skin areas and parts of the body against pressure, impacts and friction.

13. A wound dressing comprising an adhesive composition according to any one of claims 1-10.

14. A ostomy appliance comprising an adhesive composition according to any one of claims 1-10.

## Patentansprüche

1. Druckempfindliche Kleberzusammensetzung, die zum Aufbringen auf menschliche oder tierische Haut geeignet ist und enthält:
a) einen in Wasser dispergierbaren Polyester,
b) ein oder mehrere Polymere, die unter Polyolefinen oder Copolymeren oder Polymerblends daraus ausgewählt sind, und/oder ein oder mehrere physikalisch vernetzte Elastomere, die unter Blockcopolymeren, die aus Styrol und einem oder mehreren Olefinen bestehen, ausgewählt sind, und/oder ein oder mehrere Polyethylenglykole und/oder Glycerin,
c) wahlweise ein Buten-Polymer oder Buten-Copolymer,
d) wahlweise ein klebrigmachendes Harz, das teilweise oder vollständig hydriert sein kann,
e) wahlweise ein mikrokristallines Wachs,
f) wahlweise einen Weichmacher,
g) ein oder mehrere Hydrokolloide und
h) wahlweise ein Pigment.

2. Druckempfindliche Kleberzusammensetzung nach Anspruch 1, **dadurch gekennzeichnet, dass** sie enthält:
einen in Wasser dispergierbaren Polyester in einem Mengenanteil von 10-50 %,
ein oder mehrere Polymere, die unter Polyolefinen oder Copolymeren oder Polymerblends daraus ausgewählt sind, und/oder ein oder mehrere physikalisch vernetzte Elastomere, die unter Blockcopolymeren, die aus Styrol und einem oder mehreren Olefinen bestehen, ausgewählt sind, und/oder ein oder mehrere Polyethylenglykole und/oder Glycerin in einem Mengenanteil von 10-70 %,
ein Buten-Polymer oder Buten-Copolymer in einem Mengenanteil von 0-15 %,
ein klebrigmachendes Harz, das teilweise oder vollständig hydriert sein kann, in einem Mengenanteil von 0-30 %,
ein mikrokristallines Wachs in einem Mengenanteil von 0-10 %,
einen Weichmacher in einem Mengenanteil von 0-10 %,
ein oder mehrere Hydrokolloide in einem Mengenanteil von bis zu 50 % und
ein Pigment in einem Mengenanteil von 0-5 %.

3. Kleberzusammensetzung nach Anspruch 1 oder 2, **dadurch gekennzeichnet, dass** das in Wasser dispergierbare Polymer ein in Wasser dispergierbarer verzweigter Polyester ist.

4. Kleberzusammensetzung nach Anspruch 3, **dadurch gekennzeichnet, dass** der verzweigte Polyester eine Brookfield-Thermosel-Viskosität bei 177 °C von 1.000-500.000 aufweist.

5. Kleberzusammensetzung nach einem der vorhergehenden Ansprüche, bei der das eine oder die mehreren Hydrokolloide unter natürlich vorkommenden Hydrokolloiden, halbsynthetischen Hydrokolloiden und synthetischen Hydrokolloiden ausgewählt sind.

6. Kleberzusammensetzung nach Anspruch 5, bei der das eine oder die mehreren Hydrokolloide ausgewählt sind unter Guargummi, Johannisbrotkerngummi, Pektin, Alginaten, Gelatine, Xanthangummi, Karayagummi, Cellulosederivaten, Natriumstärkeglykolat, Polyvinylalkohol, Polyethylenglykol und Polyacrylaten.

7. Kleberzusammensetzung nach einem der vorhergehenden Ansprüche, die einen verzweigten in Wasser dispergierbaren Polyester sowie amorphe Poly-alpha-olefine enthält und folgende Zusammensetzung aufweist:
0-10 % Ethylen-1-Buten-Copolymer;
20-40 % Propen-1-Hexen-Copolymer;
0-10 % Triblockcopolymer;
0-15 % Polybuten;
0-15 % hydrierter Poly(Kohlenwasserstoff/Terpen)-Klebrigmacher;
0-15 % gegebenenfalls hydrierte Harzsäure und Ester davon;
0-10 % Weichmacher;
10-50 % in Wasser dispergierbarer verzweigter Polyester von Eastman;
bis zu 50 % Hydrokolloide und
0-5 % Pigment.

8. Kleberzusammensetzung nach einem der Ansprüche 1 bis 6, die einen in Wasser dispergierbaren verzweigten Polyester sowie Blockcopolymere enthält und folgende Zusammensetzung aufweist:
0-10 % Triblockcopolymer;
10-50 % Diblockcopolymer;
0-10 % Weichmacher;
0-15 % Klebrigmacher;
0-10 % mikrokristallines Wachs;
10-50 % in Wasser dispergierbarer verzweigter Polyester von Eastman;
bis zu 50 % Hydrokolloide und
0-5 % Pigment.

9. Kleberzusammensetzung nach einem der Ansprüche 1 bis 6, die einen in Wasser dispergierbaren verzweigten Polyester sowie Glycerin enthält und folgende Zusammensetzung aufweist:
10-50 % Glycerin;
10-50 % in Wasser dispergierbarer verzweigter Polyester von Eastman;
bis zu 50 % Hydrokolloide und
0-5 % Pigment.

10. Kleberzusammensetzung nach einem der Ansprüche 1 bis 6, die einen in Wasser dispergierbaren verzweigten Polyester sowie Polyethylenglykol enthält und folgende Zusammensetzung aufweist:
10-50 % Polyethylenglykol;
10-50 % in Wasser dispergierbarer verzweigter Polyester von Eastman;
bis zu 50 % Hydrokolloide und
0-5 % Pigment.

11. Verfahren zur Herstellung einer druckempfindlichen Kleberzusammensetzung nach einem der Ansprüche 1 bis 10, **dadurch gekennzeichnet, dass** das eine oder die mehreren Polymeren gemischt werden, worauf der in Wasser dispergierbare Polyester zugemischt und bis zur Homogenität eingemischt wird, gegebenenfalls ein Weichmacher zugemischt wird, und ein oder mehrere Hydrokolloide unter vermindertem Druck zugemischt werden, wonach der Klebstoff aus dem Mischer entnommen und wahlweise zwischen zwei Blättern Trennpapier zu 1 mm dünnen Platten gepresst wird.

12. Verwendung einer druckempfindlichen Kleberzusammensetzung nach einem der Ansprüche 1 bis 10 zur Befestigung und Abdichtung um Stomaverbände herum, zur Befestigung von Wundverbänden, zur Befestigung von Vorrichtungen zum Sammeln von Urin, Wunddrainageverbänden, Orthosen und Prothesen sowie zum Schutz von Hautbereichen und Teilen des Körpers gegen Druck, Stoß und Reibung.

13. Wundverband, der eine Kleberzusammensetzung nach einem der Ansprüche 1 bis 10 aufweist.

14. Stomavorrichtung, die eine Kleberzusammensetzung nach einem der Ansprüche 1 bis 10 aufweist.

## Revendications

1. Composition adhésive sensible à la pression appropriée à l'application sur la peau humaine ou animale comprenant a) un polyester hydrodispersible, b) un ou plusieurs polymères choisis dans le groupe constitué des polyoléfines ou copolymères ou mélanges de ceux-ci, et/ou un ou plusieurs élastomères réticulés physiquement, choisis parmi les copolymères blocs comprenant du styrène et une ou plusieurs oléfines, et/ou un ou plusieurs polyéthylène glycols et/ou glycérol ; c) éventuellement un polymère ou copolymère de butène, d) éventuellement une résine adhésive qui peut être partiellement ou totalement hydrogénée, e) éventuellement une cire microcristalline, f) éventuellement un plastifiant, g) un ou plusieurs hydrocolloïdes et h) éventuellement un pigment.

2. Composition adhésive sensible à la pression comme revendiquée dans la revendication 1, **caractérisée en ce qu'**elle comprend de 10 à 50 % d'un polyester hydrodispersible, 10 à 70 % d'un ou de plusieurs polymères choisis dans le groupe constitué des polyoléfines ou copolymères ou mélanges de celles-ci, et/ou un ou plusieurs élastomères réticulés physiquement, choisis parmi les copolymères blocs comprenant du styrène et une ou plusieurs oléfines, et/ou un ou plusieurs polyéthylène glycols et/ou glycérol ; 0 à 15 % d'un polymère ou copolymère de butène, 0 à 30 % d'une résine adhésive qui peut être partiellement ou totalement hydrogénée, 0 à 10 % d'une cire microcristalline, 0 à 10 % de plastifiant, jusqu'à 50 % d'un ou de plusieurs hydrocolloïdes et 0 à 5 % de pigment.

3. Composition adhésive comme revendiquée dans la revendication 1 ou 2, **caractérisée en ce que** le polymère hydrodispersible est un polyester ramifié hydrodispersible.

4. Composition adhésive comme revendiquée dans la revendication 3, **caractérisée en ce que** le polyester ramifié possède une viscosité de Brookfield Thermosel à 177° C de 1000 à 500 000.

5. Composition adhésive comme revendiquée dans l'une quelconque des revendications précédentes, dans laquelle le seul ou les multiples hydrocolloïdes sont choisis parmi les hydrocolloïdes naturels, les hydrocolloïdes semi-synthétiques et les hydrocolloïdes synthétiques.

6. Composition adhésive comme revendiquée dans la revendication 5, dans laquelle le seul ou les multiples hydrocolloïdes sont choisis parmi la gomme guar, la gomme de graines de caroube, la pectine, les alginates, la gélatine, la gomme xanthane, la gomme karaya, les dérivés de cellulose, le glycolate d'amidon sodique, l'alcool polyvinylique, le polyéthylène glycol et les polyacrylates.

7. Composition adhésive comme revendiquée dans l'une quelconque des revendications précédentes, qui comprend un polyester hydrodispersible ramifié et des poly-alpha-oléfines amorphes et possède la composition suivante :
0 à 10 % de copolymère éthylène/ 1-butène ;
20 à 40 % de copolymère propène/ 1-hexène ;
0 à 10 % de copolymère tribloc ;
0 à 15 % de polybutène ;
0 à 15 % d'adhésif polyterpène liquide ;
0 à 15 % d'adhésif poly(hydrocarbure/terpène) hydrogéné ;
0 à 15 % éventuellement d'acide de colophane hydrogéné et de ses esters ;
0 à 10 % de plastifiant ;
10 à 50 % de polyester ramifié hydrodispersible Eastman ;
jusqu'à 50 % d'hydrocolloïdes ; et
0 à 5 % de pigment.

8. Composition adhésive comme revendiquée dans l'une quelconque des revendications 1 à 6, qui comprend un polyester hydrodispersible ramifié et des copolymères blocs et possède la composition suivante:
0 à 10 % de copolymère tribloc ;
10 à 50 % de copolymère dibloc ;
0 à 10 % de plastifiant ;
0 à 15 % d'adhésif ;
0 à 10 % de cire microcristalline ;
10 à 50 % de polyester ramifié hydrodispersible Eastman ;
jusqu'à 50 % d'hydrocolloïdes ; et
0 à 5 % de pigment.

9. Composition adhésive comme revendiquée dans l'une quelconque des revendications 1 à 6, qui comprend un polyester hydrodispersible ramifié et du glycérol et possède la composition suivante:
10 à 50 % de glycérol ;
10 à 50 % de polyester ramifié hydrodispersible Eastman ;
jusqu'à 50 % d'hydrocolloïdes ; et
0 à 5 % de pigment.

10. Composition adhésive comme revendiquée dans l'une quelconque des revendications 1 à 6, qui comprend un polyester hydrodispersible ramifié et du polyéthylène glycol et possède la composition :
10 à 50 % de polyéthylène glycol ;
10 à 50 % de polyester ramifié hydrodispersible Eastman ;
jusqu'à 50 % d'hydrocolloïdes ; et
0 à 5 % de pigment.

11. Procédé de préparation d'une composition adhésive sensible à la pression, comme définie dans l'une quelconque des revendications 1 à 10, **caractérisé en ce qu'**un ou plusieurs polymères sont mélangés, après quoi le polyester hydrodispersible est additionné et mélangé jusqu'à homogénéité, un plastifiant est éventuellement ajouté et un ou plusieurs hydrocolloïdes sont additionnés sous pression réduite, après quoi l'agent adhésif est retiré du mélangeur et éventuellement comprimé en plaques minces de 1 mm d'épaisseur entre deux feuilles de papier détachable.

12. Utilisation d'une composition adhésive sensible à la pression, définie comme définie dans l'une quelconque des revendications 1 à 10, pour fixer des pansements pour stomie et rendre étanche leur périphérie, pour fixer des pansements cicatrisants, pour fixer des dispositifs destinés à la collecte de l'urine, des pansements de drainage des plaies, des orthoses et des prothèses et pour protéger les zones cutanées et parties du corps contre la pression, les chocs et la friction.

13. Pansement cicatrisant comprenant une composition adhésive conformément à l'une quelconque des revendications 1 à 10.

14. Dispositif pour stomie comprenant une composition adhésive conformément à l'une quelconque des revendications 1 à 10.
